# FASCICULE DE BREVET EUROPEEN

(11) **EP 3 413 931 B1**
(45) Date de publication et mention de la délivrance du brevet: **02.12.2020**
(21) Numéro de dépôt: 17707642.9
(22) Date de dépôt: 07.02.2017
(51) Int. Cl.: A61L 9/14, A01M 1/20

(54) **APPAREIL DE TRAITEMENT D'UNE ATMOSPHÈRE**
VORRICHTUNG ZUR BEHANDLUNG EINER UMGEBUNG
DEVICE FOR TREATING AN ENVIRONMENT

(30) Priorité: 08.02.2016 FR 1600208
(43) Date de publication de la demande: 19.12.2018
(73) Titulaire: Octopus Robots, 49300 Cholet (FR)
(72) Inventeur: SOMVILLE, Olivier, 49300 Cholet (FR)
(74) Mandataire: Fédit-Loriot
(86) Numéro de dépôt international: PCT/FR2017/050277
(87) Numéro de publication internationale: WO 2017/137693

(56) Documents cités:
- WO-A1-2007/125100
- FR-A1- 2 216 030
- US-A1- 2008 223 953
- US-A1- 2011 114 744

## Description

La présente invention concerne le domaine des dispositifs de traitement d'une atmosphère.

Dans le cadre des technologies d'assainissement d'une atmosphère impliquant la diffusion d'un agent d'assainissement en solution sous forme finement divisée dans l'atmosphère à assainir, l'invention propose de provoquer la division d'une telle solution par effet piézoélectrique. Alors que suivant la technique que l'on utilise actuellement couramment, on pulvérise la solution en l'entraînant dans un jet d'air qui la projette en gouttelettes pour créer un brouillard, l'invention prévoit de provoquer la brumisation de la solution en la faisant passer sur des membranes piézoélectriques sollicitées en vibration pour créer une pluralité de masses de brouillard.

Cette technique, connue en elle-même sous les notions de brumisation ou de nébulisation, permet de produire un brouillard de particules liquides en suspension dans l'air environnant au voisinage de la membrane piézoélectrique. Suivant l'invention, c'est ce brouillard que l'on entraîne à distance de la membrane dans l'atmosphère à assainir. Il suffit d'un courant de ventilation à faible vitesse renouvelant l'environnement de la membrane, que l'on peut ensuite guider en répartition jusque dans l'atmosphère à traiter en assainissement. Il n'y a nul besoin des grandes vitesses qui sont nécessaires dans les techniques de pulvérisation par projection, et la diffusion du produit de traitement aux alentours s'effectue sans ébullition ni turbulences.

L'invention propose plus spécifiquement un appareil d'assainissement d'une atmosphère à traiter, dans lequel on produit un brouillard assainissant en soumettant un liquide assainissant aux vibrations de membranes piézoélectriques, en véhiculant différentes masses de brouillard ainsi produites par une pluralité de courants d'air individualisés qui les entraînent respectivement à l'écart des membranes en les guidant individuellement vers un endroit spécifique de l'atmosphère à traiter.

En pratique on utilise de préférence dans ledit appareil une batterie de brumisation à membranes piézoélectriques.

Avantageusement selon l'appareil de l'invention, on alimente les différentes membranes en continu en liquide assainissant en assurant la recirculation du liquide non consommé dans la production du brouillard.

De préférence selon l'invention, on alimente les membranes piézoélectriques en canalisant le liquide assainissant au sein de différentes cuvettes au fond desquelles lesdites membranes sont respectivement présentes.

De préférence selon l'invention, on assure la recirculation du liquide non consommé dans la formation du brouillard, par débordement dudit liquide hors desdites cuvettes vers un réservoir d'alimentation contenant du liquide assainissant.

De préférence selon l'invention, on contrôle la quantité de liquide consommée pour la production du brouillard au cours du temps.

Le contrôle de cette quantité de liquide consommée en combinaison avec la mise en recirculation du liquide non consommé permet de connaître la quantité de brouillard diffusée dans l'atmosphère à traiter et ainsi de s'assurer qu'elle est suffisante pour assainir l'atmosphère.

Avantageusement selon l'invention pour entraîner les masses de brouillard, on fait circuler en circuit ouvert un flux d'air que l'on répartit pour former différents courants d'air ascendants qui entraînent les masses de brouillard vers l'atmosphère à traiter.

Les différents courants d'air sont ainsi ventilés vers des jets brouillassants produits par les différentes couches de liquide soumises aux vibrations des différentes membranes piézoélectriques, pour entraîner individuellement chaque masse de brouillard par léchage dudit jet vers une sortie qui lui est dédiée.

En outre selon un cas préféré de l'invention, on déplace les masses de brouillard guidées individuellement vers l'atmosphère par une ventilation d'air supplémentaire qui les disperse dans l'atmosphère et permet de mieux atteindre les différentes surfaces dans l'atmosphère.

Selon l'appareil de l'invention, on utilise de préférence un liquide aqueux. En particulier pour assainir l'atmosphère on utilise un liquide assainissant contenant en solution un agent assainissant choisi parmi les agents biocides et les produits phytosanitaires. Par exemple on utilise comme agent biocide le peroxyde d'hydrogène.

L'invention propose ainsi un appareil d'assainissement d'une atmosphère à traiter qui comporte des moyens de production d'un brouillard par vibration de membranes piézoélectriques, ledit appareil comportant au moins une batterie de brumisation d'un liquide assainissant équipée de membranes piézoélectriques, et des moyens pour véhiculer différentes masses du brouillard ainsi produit par une pluralité de courants d'air individualisés entraînant respectivement les différentes masses de brouillard à l'écart des membranes en les guidant individuellement vers un endroit spécifique de l'atmosphère à traiter.

De préférence l'appareil selon l'invention comporte différentes cuvettes dans lesquelles sont respectivement disposées différentes membranes piézoélectriques de ladite batterie de brumisation et en ce que lui sont associés des moyens de réglage du débit de liquide brumisé par maintien d'un niveau de liquide à hauteur constante au-dessus de la membrane correspondante, en particulier par débordement du liquide au-dessus d'une paroi de cuvette. Lesdites cuvettes sont associées à la batterie de brumisation pour produire le brouillard.

Avantageusement selon l'invention, ledit appareil comporte un bloc usiné permettant de produire des masses de brouillard individualisées. Ce bloc intègre la batterie de brumisation et y sont usinées individuellement les différentes cuvettes réceptrices des membranes piézoélectriques ainsi qu'une pluralité de cavités internes de collecte de brouillard débouchant chacune dans un conduit de guidage de brouillard ascendant vers l'atmosphère à traiter, ledit brouillard étant entraîné et guidé par un courant d'air ascendant admis dans les cavités correspondantes au-dessus desdites cuvettes.

De préférence selon l'invention, ledit appareil comporte un réservoir contenant le liquide assainissant pour alimenter les membranes piézoélectriques en liquide, par l'intermédiaire des cuvettes entourant les membranes.

De préférence un réseau de canaux usinés dans le bloc alimenté par une conduite reliée au réservoir de liquide dessert chaque membrane.

De préférence l'appareil comporte un module de pesée sensible au poids dudit réservoir et des moyens de détermination de la quantité de liquide consommée pour la production des masses de brouillard au cours du temps en fonction du poids du réservoir déterminé par ledit module de pesée.

De préférence selon l'invention, ledit appareil comporte des moyens assurant la recirculation de liquide débordant des cuvettes vers ledit réservoir, par des conduits déversoirs.

En particulier selon l'invention, l'appareil comprend des moyens d'injection d'un flux d'air dans le réservoir, ces dits moyens comportant un ventilateur situé hors dudit bloc et dudit réservoir, et des moyens de répartition de ce flux d'air vers lesdites différentes cavités de collecte de brouillard produit par les membranes dans lesdites cuvettes.

Avantageusement selon l'invention, les conduits pour le guidage individuel des différentes masses de brouillard, ont une forme coudée sur un trajet ascendant, cette forme faisant obstacle au passage des trop grosses gouttelettes de liquide, afin de produire un brouillard à sensation sèche. La forme coudée peut se présenter sous forme d'un « S ».

De préférence l'appareil selon l'invention comprend en outre des moyens supplémentaires de soufflage d'air, situés en sortie des conduits de guidage de brouillard, ces dits moyens étant disposés à l'extérieur dudit bloc et de manière à ce que les masses de brouillard sortant des conduits de guidage dans l'atmosphère ne viennent en contact direct avec ces dits moyens afin d'éviter la recombinaison des gouttelettes de brouillard entre elles.

Selon un cas particulier de l'invention, les conduits déversoirs ramenant le liquide en débordement des cuvettes sont connectés d'une part à des lumières usinées dans le bloc débouchant respectivement dans les cavités du bloc et d'autre part à la partie supérieure du réservoir, les conduits déversoirs servant en outre de moyens de répartition du flux d'air injecté dans le réservoir vers lesdites cavités.

Dans le cas particulier de liquides à propriétés tensio-actives pouvant produire de la mousse lors de leur mise en œuvre, avantageusement selon l'invention, les différentes cavités usinées au-dessus des cuvettes sont aménagées pour que les différents courants d'air ascendant d'entraînement du brouillard qui les parcourent respectivement soient déviés vers la partie ascendante du jet brouillassant jaillissant de la cuvette, évitant ainsi le contact de cet air avec le jet au moment où il vient en contact avec une paroi de la cavité, contact créant de la mousse. Une paroi intérieure pentue disposée dans le prolongement intérieur de la paroi externe du bloc usiné dévie ledit courant d'air entrant dans la cavité alors que le jet brouillassant vient retomber dans la retenue délimitée par cette paroi pentue et ladite paroi externe du bloc. Un canal de réacheminent reliant ladite retenue et la cuvette de la membrane piézoélectrique permet que le liquide recueilli dans ladite retenue soit remis en circulation. Un tel aménagement évite donc d'entraîner de la mousse dans le brouillard sortant dans l'atmosphère à traiter.

L'appareil selon l'invention peut comprendre plusieurs batteries de brumisation respectivement intégrées chacune dans un bloc usiné et chacune associée à plusieurs conduits de guidage de courants d'air ascendant, le réservoir d'alimentation en liquide assainissant étant commun auxdites batteries. Il est alors possible d'assainir une plus grande étendue et d'atteindre différentes surfaces en diffusant plus largement le brouillard assainissant, les batteries de brumisation et les conduits de guidage de brouillard étant disposés de manière adéquate.

Avantageusement selon l'invention, l'appareil peut être mobile, notamment robotisé, en ce qu'il comprend un châssis mobile portant les batteries de brumisation et des moyens de commande de ses déplacements pour lui faire parcourir la surface au sol desservant l'atmosphère à traiter par partition de cette surface en couloirs à partir d'une cartographie autonome ou de données acquises par apprentissage.

L'invention sera maintenant plus complètement décrite dans le cadre de caractéristiques préférées et de leurs avantages, en faisant référence aux figures 1 à 10 annexées dans lesquelles :
- la figure 1 illustre schématiquement un appareil de production d'un brouillard assainissant, selon une vue partielle en coupe transversale,
- la figure 1A illustre schématiquement et partiellement, selon une vue de dessus, l'appareil de la figure 1 ;
- la figure 2 illustre schématiquement en perspective et partiellement l'appareil de la figure 1 ;
- la figure 3 illustre schématiquement, selon une vue partielle en coupe longitudinale, l'appareil illustré aux figures 1 et 2 ;
- la figure 4 illustre schématiquement en coupe transversale une partie d'un appareil selon l'invention en liaison avec les figures 1 à 3 ;
- la figure 5 est illustre schématiquement en perspective de trois-quarts et en vue de dessus une partie d'un appareil selon l'invention en liaison avec les figures 1 à 4 ;
- la figure 6 illustre un appareil mobile intégrant des dispositifs tels que illustrés aux figures 1 à 5 et 7, selon une vue en perspective de trois-quarts;
- la figure 6A représente une vue en coupe transversale de l'appareil mobile illustré en figure 6;
- la figure 7 illustre schématiquement en perspective de trois-quarts une vue partielle de l'intérieur d'un dispositif en liaison avec les figures précédentes ;
- la figure 8 illustre schématiquement une variante d'un appareil de production d'un brouillard assainissant, vu partiellement en coupe transversale,
- la figure 9 illustre schématiquement et partiellement, l'appareil de la figure 8 selon une vue en éclaté et en perspective de trois-quarts ;
- la figure 10 illustre schématiquement, selon une vue partielle en coupe longitudinale, l'appareil illustré aux figures 8 et 9.

Tous les composants ne sont pas représentés à la même échelle pour la clarté des figures.

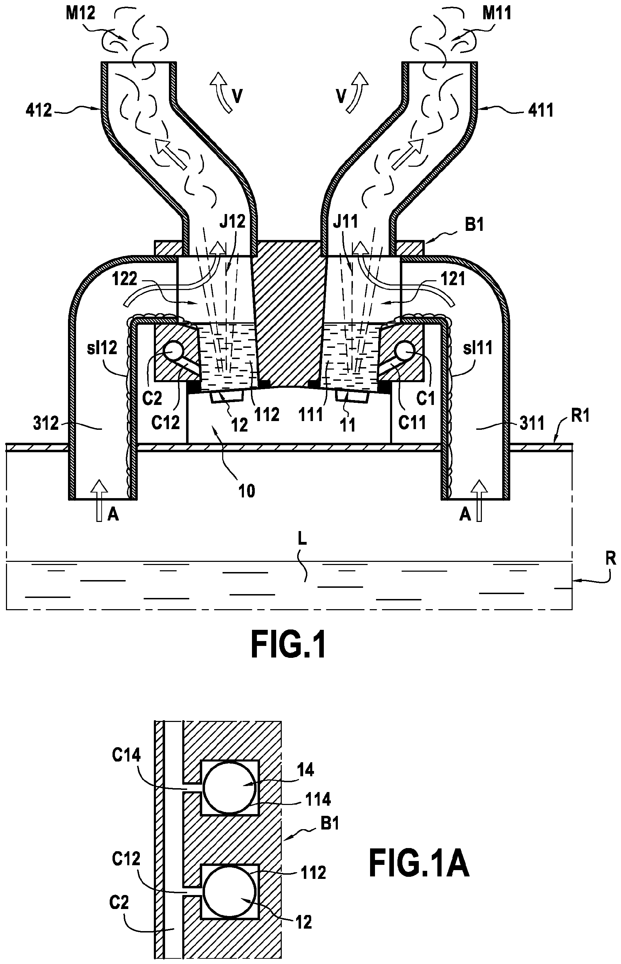

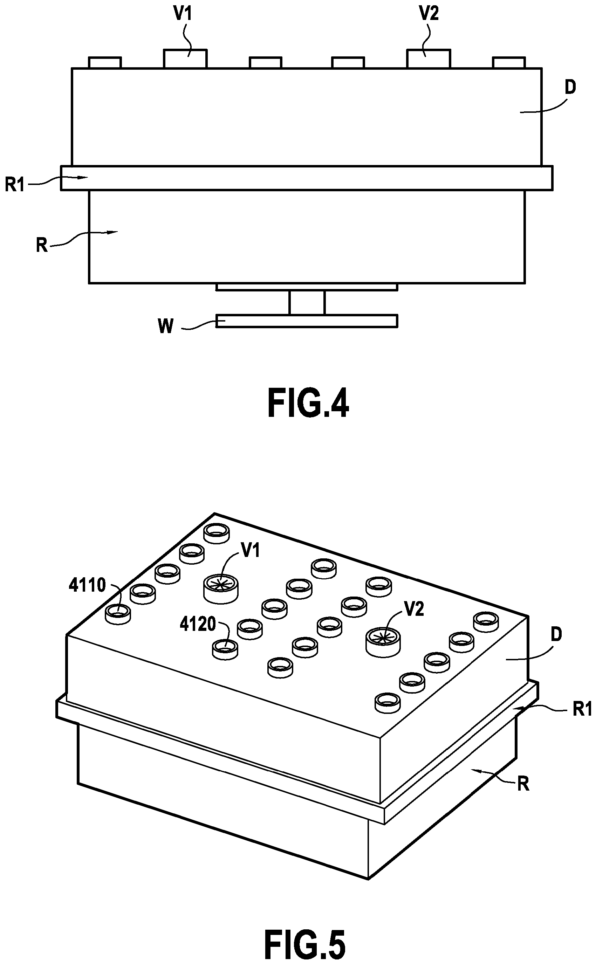

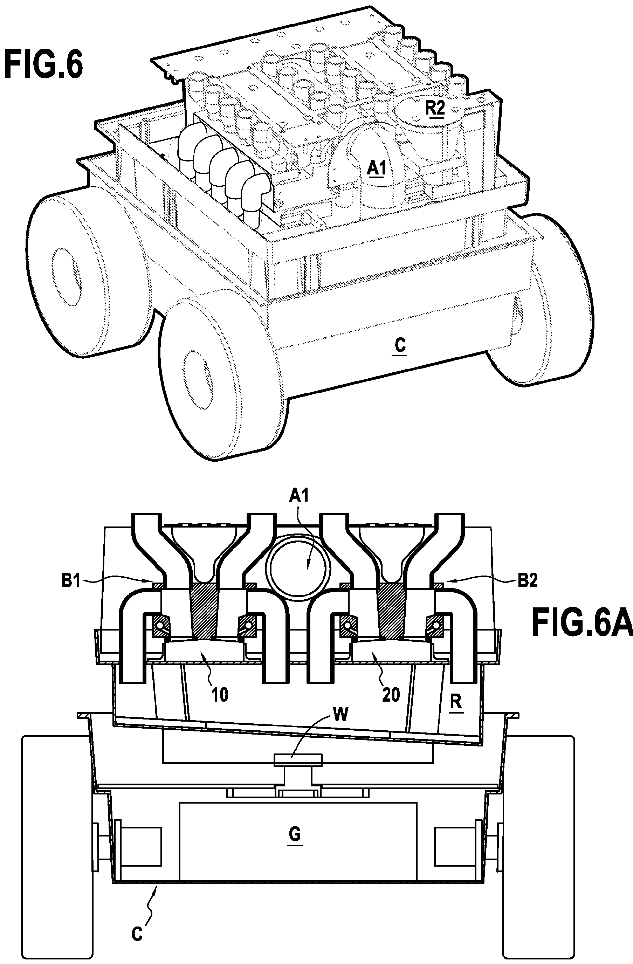

Les figures 1 à 7 illustrent un appareil de production de masses de brouillard assainissant par nébulisation d'une solution, fonctionnant selon le procédé d'assainissement d'atmosphère de l'invention, les figures 6 et 6A illustrant l'appareil monté mobile pour assainir une atmosphère.

L'appareil se présente sous forme d'un dispositif de production de masses de brouillard assainissant qui comprend une batterie de brumisation à membranes piézoélectriques 10 se présentant selon cet exemple sous forme d'une double rangée de cinq membranes piézoélectriques. Chaque membrane piézoélectrique 11, 12 est selon cet exemple une membrane en céramique, mise en vibration par raccordement de la batterie de brumisation par le cordon électrique 101 à une alimentation électrique, qui produit un jet de liquide brouillassant J11, J12 lorsqu'une solution L est soumise aux vibrations de ladite membrane.

Les vibrations des membranes piézoélectriques sont produites comme couramment à des fréquences ultrasoniques.

Selon l'invention, la batterie de membranes piézoélectriques 10 est coiffée d'un bloc B1 spécialement usiné de manière à ce que selon cet exemple chaque membrane piézoélectrique 11, 12, 14 soit individualisée au fond d'une cuvette 111, 112, 114 usinée dans ledit bloc.

Selon le mode de réalisation particulier illustré par les figures, la batterie de brumisation est recouverte par le bloc usiné, et est disposée sur le couvercle R1 d'un réservoir R contenant la solution L qui sera nébulisée par ledit dispositif pour produire une pluralité de masses de brouillard. La batterie de brumisation n'est pas immergée dans la solution à nébuliser. Cette solution peut être un liquide aqueux contenant un agent assainissant, par exemple un agent biocide, un produit phytosanitaire ou tout autre composé à diffuser dans l'atmosphère, mis en solution dans de l'eau.

La solution L dans le réservoir R est pompée, à l'aide d'une pompe P puis envoyée par une canalisation vers la batterie piézoélectrique 10 à l'aide de tuyaux de raccordement T11, T12 respectivement aux orifices O1, O2 creusés dans ledit bloc usiné B1 faisant entrée la solution vers respectivement des canaux C1, C2 usinés dans ledit bloc B1 parallèlement et respectivement à une rangée de membranes piézoélectriques, un canal C1, C2 étant relié par des conduits de dérivation respectivement aux cuvettes des membranes piézoélectriques de sa rangée, afin d'alimenter en solution chaque membrane piézoélectrique. Une certaine hauteur de liquide est maintenue au-dessus de chaque membrane céramique, qui mise en vibration, agite cette couche de liquide jusqu'à créer un jet brouillassant tel que les jets référencés J11, J12 sur la figure 1, qui jaillissent à l'aplomb des cuvettes 111,112 respectives des membranes piézoélectriques 11, 12, dans des cavités internes 121, 122 qui prolongent respectivement les cuvettes.

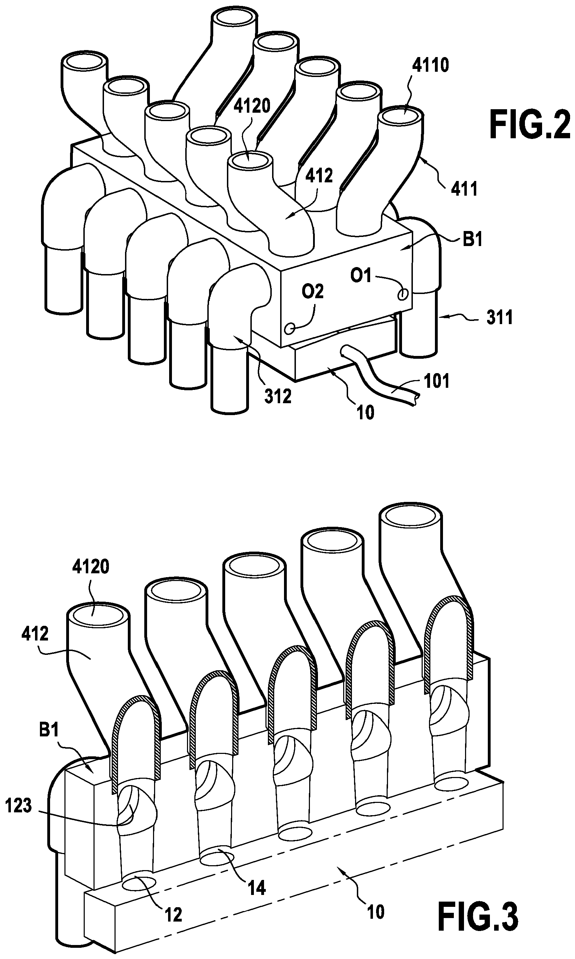

L'appareil selon l'invention prévoit que l'alimentation en liquide L de la batterie piézoélectrique se fasse en boucle, chaque membrane étant alimentée en continu par contrôle de l'apport de liquide par débordement au niveau de chaque membrane. Selon le mode de réalisation illustré aux figures 1 à 4, au niveau de chaque membrane piézoélectrique 11, 12, une lumière, telle que la lumière 123 en figure 3, est réalisée dans le bloc usiné B1 pour y insérer l'extrémité supérieure d'un conduit déversoir 311, 312 qui ramène l'excédent de liquide sl11, sl12 vers le réservoir R, ledit conduit étant connecté par son extrémité inférieure au réservoir à travers un trou traversant prévu dans le couvercle R1 du réservoir. Le niveau de liquide dans la cuvette, et donc au-dessus de la membrane, est ainsi géré par la hauteur de la paroi de cuvette et la position de cette lumière située en partie supérieure d'une paroi de référence de la cuvette.

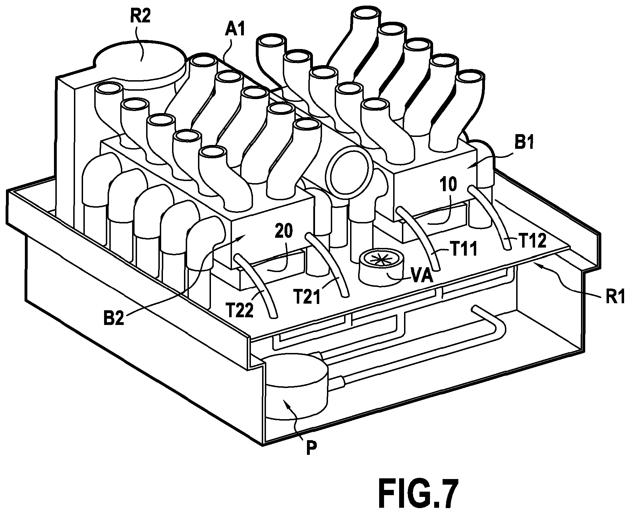

Par ailleurs, selon le mode de réalisation illustré notamment aux figures 1 à 3 et 7, le flux d'air réparti en une pluralité de courants d'air ascendants pour entraîner la masse de brouillard M11, M12 extraite par léchage du jet brouillassant J11, J12 provient d'air insufflé A depuis un ventilateur externe VA (figure 7) vers le réservoir R contenant le liquide, en partie supérieure dudit réservoir exempte de liquide. Ce flux d'air est alors réparti en différents courants d'air ascendant pour être dirigé en sortie des cuvettes, à l'aplomb des membranes, dans les cavités internes du bloc et jusque dans les conduits de guidage des masses de brouillard dans l'atmosphère à traiter. Cette répartition en courants d'air est réalisée en faisant emprunter audit flux d'air entrant chaque conduit-déversoir 311, 312 utilisé pour récupérer le surplus de liquide en débordement, comme représenté par les flèches à l'intérieur du dispositif. La quantité de liquide en surplus pour chaque membrane piézoélectrique est relativement faible de sorte qu'il reste assez d'espace dans le conduit déversoir pour que l'air passe à contre-courant du liquide en surplus.

De préférence chaque jet brouillassant J11, J12 est dirigé vers un conduit de guidage vers l'atmosphère formé selon l'exemple par un tuyau de guidage 411, 412 dont l'extrémité inférieure est connectée à un orifice de sortie de cavité interne du bloc, usiné à l'aplomb de la membrane piézoélectrique respective, de manière à ce que la masse de brouillard M11, M12 qu'entraîne le courant d'air ascendant soit dirigée vers la sortie 4110, 4120 du tuyau de guidage 411, 412 débouchant dans l'atmosphère à traiter. De préférence lesdits tuyaux de guidage 411, 412 ont une forme coudée en « S » qui permet aux trop grosses gouttes de liquide, qui pourraient condenser ensemble, de retomber dans la cuvette de la membrane piézoélectrique au lieu de s'échapper avec la masse de brouillard constituées de fines gouttelettes de solution.

L'appareil permet ainsi de créer des masses de brouillard individualisées, qui forment un brouillard assainissant « sec ». Ces masses de brouillard étant individualisées les unes par rapport aux autres, elles ne peuvent pas se recombiner pour condenser et créer un brouillard humide.

Avantageusement selon l'invention, un flux d'air additionnel, représenté par les flèches V sur la figure 1, est ventilé aux niveaux des sorties des conduits de guidage des masses de brouillard pour propulser les masses de brouillard M11, M12 sortantes, dans toute l'atmosphère à traiter, à l'aide de moyens de ventilation tels que des ventilateurs de reprise V1, V2 disposés sur la coque D recouvrant les tuyaux de guidage (voir figures schématiques 4 et 5), par exemple entre les rangées des sorties du dispositif, et de manière à ce que les masses de brouillard ne viennent pas en contact avec les ventilateurs proprement dit, évitant ainsi la recombinaison des gouttelettes de brouillard entre elles.

Pour procéder à l'assainissement d'une atmosphère, on utilise de préférence un appareil mobile tel qu'illustré aux figures 6 et 6A.

Selon cet exemple, l'appareil comprend deux batteries de membranes de brumisation à membranes piézoélectriques 10, 20, surmontées chacune d'un bloc usiné B1, B2 selon une configuration conforme à celle décrite aux figures 1 à 3, lesdites batteries de brumisation 10, 20 reposant sur le couvercle d'un même réservoir R qui contient la solution L à nébuliser. Chacune des batteries de brumisation 10, 20 est alimentée en liquide L depuis ce même réservoir R par une pompe P (voir figure schématique 7). Le réservoir est rempli en liquide L par la bouche d'alimentation R2.

L'air insufflé A dans les deux blocs usinés B1 et B2 est produit par un ventilateur commun VA (voir figure schématique 7) dont l'air généré est véhiculé par un tuyau A1 jusqu'à la partie supérieure du réservoir, exempte de liquide.

Avantageusement selon cet exemple, le réservoir R repose sur un module de pesée W, sensible au poids du réservoir, qui permet de suivre la quantité de liquide consommé pour la production des masses de brouillard au cours du temps, ce qui permet de connaître in fine le débit de brouillard et d'ainsi contrôler s'il est en adéquation avec le volume de l'atmosphère à traiter. Ce suivi permet également de prévoir quand il est nécessaire de réapprovisionner ledit dispositif en liquide assainissant.

Avantageusement le retour de l'appareil vers une base pour le recharger en liquide peut ainsi être géré par un programme informatique à bord de l'appareil ou par connexion sans fil avec un ordinateur à distance.

Le module de pesée W et au moins une partie du réservoir R sont disposés dans un châssis C, monté sur roues. Le châssis C comporte de préférence en outre une batterie électrique G, source d'énergie pour la motion de l'appareil et le fonctionnement des divers équipements nécessitant une source d'énergie tels que les membranes piézoélectriques, les ventilateurs, la pompe, les divers moyens de programmation.

Avantageusement l'appareil est programmé pour parcourir la surface au sol desservant l'atmosphère à traiter par partition de cette surface en couloirs à parcourir, à partir d'une cartographie autonome ou de données acquises par apprentissage suivant l'enregistrement d'un premier parcours réalisé par un opérateur.

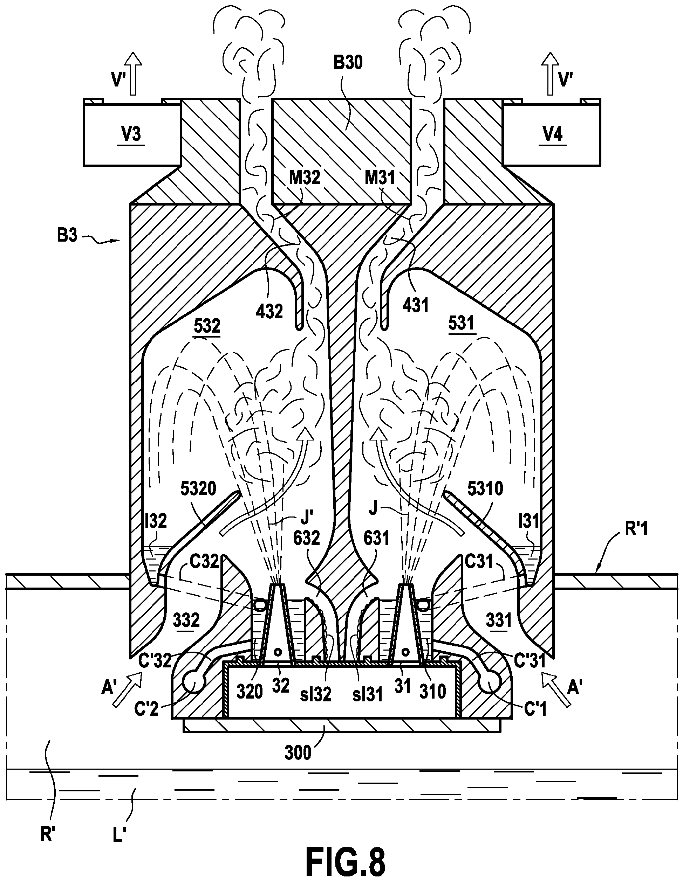

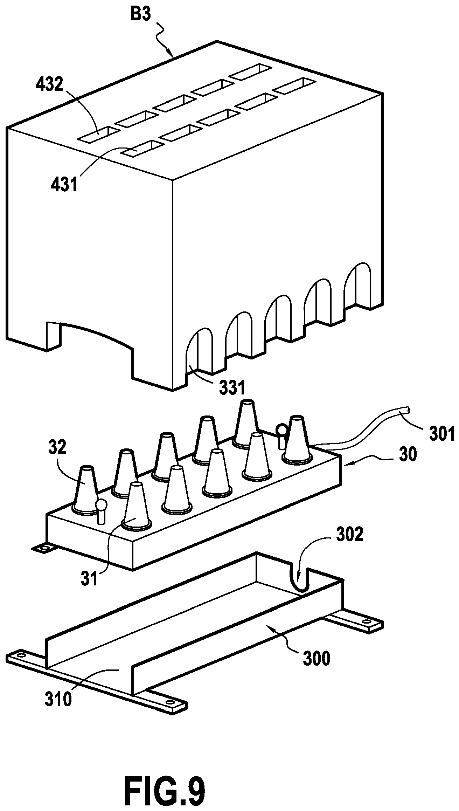

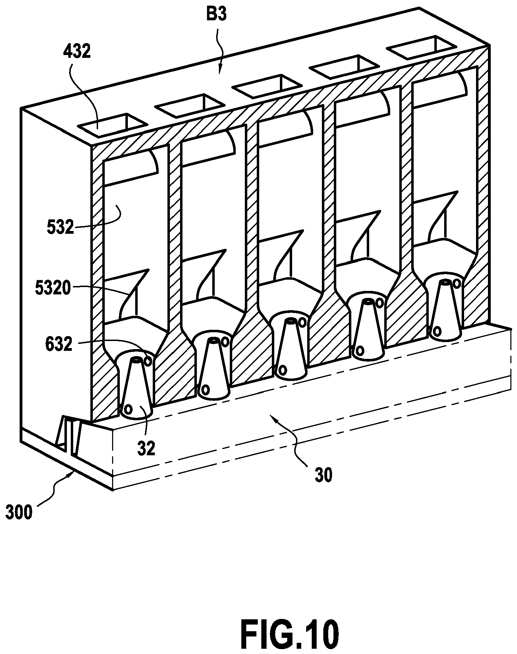

Les figures 8 à 10 illustrent une variante d'un appareil de nébulisation d'une solution pour la production de masses de brouillard assainissant. Cet appareil est plus particulièrement adapté aux solutions à nébuliser qui ont des propriétés tensio-actives qui risquent d'entraîner la formation de mousse lors de leur mise en œuvre.

Comme pour l'appareil de production de masses de brouillard précédemment décrit, on utilise une batterie de brumisation à membranes piézoélectriques surmontée d'un bloc usiné qui permet d'avoir différentes membranes piézoélectriques respectivement disposées au sein de différentes cuvettes afin de produire différentes masses de brouillard.

Le bloc usiné B3 de l'appareil selon cet exemple est réalisé de sorte que les jets brouillassants produits par les différentes couches de liquide mises en vibration par les membranes piézoélectriques viennent frapper une paroi à l'écart des courants d'air ascendants qui pourraient entraîner avec le brouillard, la mousse qui se forme lorsque le jet vient frapper les parois internes du bloc.

L'appareil selon cet exemple comprend une batterie de brumisation 30 qui se présente selon cet exemple sous forme d'une double rangée de cinq éléments piézoélectriques, chacun formé d'une membrane en céramique, mise en vibrations par une alimentation électrique, et surmontée d'un cône, ouvert en sa base et son sommet, pour diriger le jet de liquide brouillassant, dont émane le brouillard, qui se forme lors de la nébulisation d'une solution.

Le bloc B3 est usiné intérieurement de manière à ce que chaque membrane piézoélectrique 31, 32 soit individualisé au fond d'une cuvette 310, 320 qui reçoit le liquide à nébuliser depuis un réservoir R' et acheminer par une canalisation jusqu'aux orifices d'entrée du liquide creusés dans le bloc B3. Ces orifices sont l'entrée respective de canaux C'1 et C'2 creusés symétriquement dans le bloc B3, parallèlement et respectivement à une rangée de membranes piézoélectriques, pour alimenter en liquide respectivement les rangées de membranes piézoélectriques, le liquide empruntant une dérivation individualisée C'31, C'32 débouchant dans la cuvette qui entoure l'élément piézoélectrique considéré, le liquide pénétrant jusqu'à la membrane piézoélectrique par des orifices à travers le cône surmontant ladite membrane. Le niveau de liquide au-dessus de la membrane est géré par débordement du liquide au-dessus d'une paroi de la cuvette dont la hauteur fixe ce niveau, le liquide en débordement sl31, sl32 dans cet exemple s'écoulant par une gorge formant déversoir 631, 632 creusée dans le bloc usiné vers la partie centrale de la batterie piézoélectrique pour retomber dans le support 300 de la batterie piézoélectrique, exempt de paroi montante sur l'un de ses côtés transverses 310 pour permettre l'écoulement dudit liquide en surplus vers le réservoir R'. Ce support 300 étant disposé en partie haute du réservoir R', au-dessus du niveau le plus haut de liquide, le liquide débordant retombe ainsi dans le réservoir R' sans toutefois que la batterie de brumisation soit immergée dans la solution à nébuliser contenue dans ledit réservoir. Le réservoir R' est en outre fermé par un couvercle R'1 dans lequel est inséré la partie inférieure du bloc usiné B3.

La couche de liquide au-dessus de chaque membrane 31, 32 en vibration est nébulisée en produisant un jet de liquide brouillassant J, J', dont émane le brouillard, qui jaillit du cône surmontant la membrane dans une cavité 531,532 creusée dans le bloc usiné B3.

Un flux d'air ventilé, représenté par les flèches A' en figure 8, se répartit en courants d'air ascendants en entrant dans les bouches d'entrée 331, 332 creusées dans la paroi externe du bloc B3, débouchant respectivement au niveau de chaque élément piézoélectrique 31, 32,est dévié par une paroi pentue 5310, 5320 présente dans la cavité 531, 532 creusée dans le bloc, en prolongement de la surface interne de la paroi externe du bloc afin d'orienter cet air vers la partie intérieure de la cavité où le jet brouillassant est ascendant et vers le conduit de guidage de la masse de brouillard M31, M32 qu'extrait ledit air par léchage du jet brouillassant. Le courant d'air entraîne ainsi le brouillard au niveau de la partie ascendante du jet, avant que ledit jet entre en contact avec une paroi interne du bloc pour créer de la mousse. Le brouillard est ainsi extrait du jet sans mousse.

Le jet brouillassant retombe de côté en venant heurter les parois de la cavité qui sont hors du trajet ascendant du courant d'air, le liquide du jet non évacué sous forme de brouillard se trouve alors contenu dans la retenue formée par l'espace délimité par cette même paroi pentue et la paroi externe dudit bloc. Le liquide recueilli l31, l32 dans cette retenue est réacheminé vers la cuvette de l'élément piézoélectrique considéré 31, 32 via un canal C31, C32 creusé dans ledit bloc reliant ladite retenue et la cuvette.

La paroi pentue 5310, 5320 joue le rôle de déflecteur pour le courant d'air entrant dans la cavité et permet de retenir le liquide avec la mousse.

La masse de brouillard M31, M32 est entraînée sans mousse par le courant d'air ascendant qui parcourt sa cavité 531, 532 du bloc B3 et guidée dans le conduit de guidage 431, 432 du bloc usiné B3, jusqu'à la lumière creusée dans la paroi supérieure dudit bloc connectée selon cet exemple avec un bloc additionnel B30 disposé en prolongement du bloc principal B3. Ce bloc additionnel B30 prolonge le conduit de guidage de la masse de brouillard de sorte que le conduit de guidage complet de la masse de brouillard a une forme coudée, en « S », sur son trajet ascendant, pour retenir les trop grosses gouttes de liquide présentes dans le jet de brouillard.

En sorties du bloc additionnel, sont disposés des ventilateurs de reprises V3, V4 qui génèrent un flux d'air V' supplémentaire aidant à diffuser les masses de brouillard sortantes dans l'atmosphère à traiter. Ces ventilateurs de reprise sont disposées juste en dessous des sorties du brouillard de manière à ce que les masses de brouillard n'aient pas à les traverser au risque de provoquer leur condensation.

Comme pour l'exemple précédent décrit aux figures 1 à 7, l'appareil permet de créer différentes masses de brouillard, au niveau de chaque membrane piézoélectrique, qui ne peuvent pas se recombiner pour condenser, et forment ainsi un brouillard assainissant «à sensation sèche ».

L'appareil illustré aux figures 8 à 10 peut être monté en plusieurs exemplaires disposés symétriquement sur un châssis mobile de manière similaire à celui décrit aux figures 6 et 6A, en utilisant un seul réservoir R', le montage étant adapté aux caractéristiques de cette variante de dispositif.

Il ressort néanmoins de ce qui précède que l'invention n'est pas limitée aux modes de mise en œuvre qui ont été spécifiquement décrits et représentés sur les figures. L'appareil peut être utilisé pour traiter une atmosphère à assainir, tout environnement incluant toutes surfaces présentes et accessibles dans ce lieu, par exemple par l'emploi d'un agent biocide mis en solution en milieu aqueux, de même pour diffuser un agent phytosanitaire sur des plantes, notamment dans une serre, ou tout autre agent actif.

L'appareil peut être équipé d'une seule ou de plusieurs batteries de brumisation à membranes piézoélectriques.

## Revendications

1. Appareil d'assainissement d'une atmosphère à traiter comportant des moyens de production d'un brouillard par vibration de membranes piézoélectriques, ledit appareil comportant au moins une batterie de brumisation (10) d'un liquide assainissant équipée de membranes piézoélectriques (11, 12), et des moyens pour véhiculer différentes masses du brouillard ainsi produit par une pluralité de courants d'air individualisés entraînant respectivement les différentes masses de brouillard à l'écart des membranes en les guidant individuellement vers un endroit spécifique de l'atmosphère à traiter ; ledit appareil comportant différentes cuvettes dans lesquelles sont respectivement disposées différentes membranes piézoélectriques de ladite batterie de brumisation et en ce que lui sont associés des moyens de réglage du débit de liquide brumisé par maintien d'un niveau de liquide à hauteur constante au-dessus de la membrane correspondante, ledit appareil comportant un bloc (B1) intégrant ladite batterie de brumisation et dans lequel sont usinées individuellement les différentes cuvettes (111, 112) réceptrices des membranes piézoélectriques ainsi qu'une pluralité de cavités internes (121, 122) de collecte de brouillard débouchant chacune dans un conduit de guidage de brouillard ascendant vers l'atmosphère à traiter par un flux d'air admis dans les cavités correspondantes au-dessus desdites cuvettes, **caractérisé en ce que** ledit appareil comprend un réservoir (R, R') contenant le liquide d'assainissement et des moyens assurant la recirculation vers ledit réservoir de liquide débordant des cuvettes par des conduits déversoirs (311, 312, 631, 632) ; l'appareil comprenant un réservoir (R, R') contenant le liquide d'assainissement, des moyens d'injection d'un flux d'air dans ledit réservoir qui comportent un ventilateur (VA) situé hors dudit bloc et dudit réservoir, et des moyens de répartition de ce flux d'air (311, 312 ; 331, 332) vers lesdites différentes cavités de collecte de brouillard produit par les membranes dans lesdites cuvettes; lesdits conduits déversoirs (311, 312) étant connectés d'une part à des lumières (123) usinées dans ledit bloc débouchant respectivement dans lesdites cavités et d'autre part à la partie supérieure dudit réservoir, lesdits conduits déversoirs servant en outre de moyens de répartition dudit flux d'air injecté dans le réservoir.

2. Appareil selon la revendication 1, **caractérisé en ce que** lesdits conduits de guidage (411, 412 ; 431, 432) pour le guidage individuel des différentes masses de brouillard, ont une forme coudée sur un trajet ascendant.

3. Appareil selon la revendication 1 ou 2, **caractérisé en ce qu'**il comprend en outre des moyens supplémentaires de soufflage d'air (V1, V2 ; V3, V4) en sortie desdits conduits de guidage de brouillard, lesdits moyens étant disposés à l'extérieur dudit bloc et de manière à ce que les masses de brouillard sortant desdits conduits ne viennent en contact direct avec ces dits moyens.

4. Appareil selon l'une des revendications 1 à 3, **caractérisé en ce qu'**il comprend un réservoir (R, R') contenant le liquide d'assainissement et un module de pesée (W) sensible au poids dudit réservoir et des moyens de détermination de la quantité de liquide consommée pour la production des masses de brouillard au cours du temps en fonction du poids du réservoir déterminé par ledit module de pesée.

5. Appareil selon l'une des revendications 1 à 4, **caractérisé en ce qu'**il comprend plusieurs batteries de brumisation respectivement intégrées chacune dans un bloc usiné et chacune associée à plusieurs conduits de guidage de courants d'air ascendant, et un réservoir d'alimentation en liquide assainissant commun auxdites batteries.

6. Appareil selon la revendication 5, comprenant un châssis mobile (C) portant lesdites batteries de brumisation et des moyens de commande de ses déplacements pour lui faire parcourir la surface au sol desservant l'atmosphère à traiter par partition de cette surface en couloirs à partir d'une cartographie autonome ou de données acquises par apprentissage.

## Patentansprüche

1. Vorrichtung zur Reinigung einer zu behandelnden Umgebung, umfassend Mittel zur Erzeugung eines Nebels mittels Vibration von piezoelektrischen Membranen, wobei die Vorrichtung mindestens eine Batterie (10) zur Vernebelung einer Reinigungsflüssigkeit umfasst, die ausgestattet ist mit piezoelektrischen Membranen (11, 12) und Mitteln zur Förderung von verschiedenen Massen des so produzierten Nebels mittels einer Vielzahl von vereinzelten Luftströmen, die die verschiedenen Nebelmassen jeweils von den Membranen verdrängen, indem sie einzeln zu einer bestimmten Stelle der zu behandelnden Umgebung geführt werden; wobei die Vorrichtung verschiedene Küvetten umfasst, in denen jeweils verschiedene piezoelektrische Membranen der Batterie zur Vernebelung angeordnet sind, und wobei ihr Mittel zur Einstellung der Durchflussmenge der vernebelten Flüssigkeit, durch Beibehalten eines Flüssigkeitsstands auf konstanter Höhe über der entsprechenden Membran, zugeordnet sind, wobei die Vorrichtung einen Block (B1) umfasst, der die Batterie zur Vernebelung integriert und in den einzeln die verschiedenen Küvetten (111, 112), die die piezoelektrischen Membranen aufnehmen, sowie eine Vielzahl von inneren Hohlräumen (121, 122) zum Sammeln von Nebel eingearbeitet sind, die jeweils in einen Kanal zur Führung von aufsteigendem Nebel in Richtung der zu behandelnden Umgebung, durch einen Luftstrom, der in die entsprechenden Hohlräume über den Küvetten zugeführt wird, münden, **dadurch gekennzeichnet, dass** die Vorrichtung einen Behälter (R, R'), der die Reinigungsflüssigkeit enthält, und Mittel umfasst, die die Rückführung von Flüssigkeit, die aus den Küvetten überläuft, mittels Überlaufkanälen (311, 312, 631, 632) zu dem Behälter sicherstellen; wobei die Vorrichtung einen Behälter (R, R'), der die Reinigungsflüssigkeit enthält, Mittel zum Einblasen eines Luftstroms in den Behälter, die einen Ventilator (VA) umfassen, der sich außerhalb des Blocks und des Behälters befindet, und Mittel zur Verteilung dieses Luftstroms (311, 312; 331, 332) in Richtung der verschiedenen Hohlräume zum Sammeln von Nebel umfasst, der von den Membranen in den Küvetten erzeugt wird; wobei die Überlaufkanäle (311, 312) zum einen mit Öffnungen (123), die in den Block eingearbeitet sind, die jeweils in die Hohlräume münden, und zum anderen mit dem oberen Teil des Behälters verbunden sind, wobei die Überlaufkanäle ferner als Mittel zur Verteilung des Luftstroms dienen, der in den Behälter eingeblasen wird.

2. Vorrichtung nach Anspruch 1, **dadurch gekennzeichnet, dass** die Führungskanäle (411, 412; 431, 432) zur individuellen Führung der verschiedenen Nebelmassen in Aufwärtsrichtung eine abgewinkelte Form aufweisen.

3. Vorrichtung nach Anspruch 1 oder 2, **dadurch gekennzeichnet, dass** sie ferner zusätzliche Luftblasmittel (V1, V2; V3, V4) am Ausgang der Kanäle zur Nebelführung umfasst, wobei die Mittel außerhalb des Blocks und derart angeordnet sind, dass die Nebelmassen, die aus den Kanälen austreten, nicht in direkten Kontakt mit diesen Mitteln kommen.

4. Vorrichtung nach einem der Ansprüche 1 bis 3, **dadurch gekennzeichnet, dass** sie einen Behälter (R, R'), der die Reinigungsflüssigkeit enthält, und ein Wiegemodul (W), das gegenüber dem Gewicht des Behälters empfindlich ist, und Mittel zur Bestimmung der Menge an Flüssigkeit, die im Laufe der Zeit für die Erzeugung der Nebelmassen verbraucht wird, in Abhängigkeit des Gewichts des Behälters umfasst, das von dem Wiegemodul bestimmt wird.

5. Vorrichtung nach einem der Ansprüche 1 bis 4, **dadurch gekennzeichnet, dass** sie mehrere Batterien zur Vernebelung, die jeweils in einen bearbeiteten Block integriert sind und die jeweils mehreren Kanälen zur Führung von aufsteigenden Luftströmen zugeordnet sind, und einen gemeinsamen Reinigungsflüssigkeitsvorratsbehälter der Batterien umfasst.

6. Vorrichtung nach Anspruch 5, umfassend ein bewegliches Chassis (C), das die Batterien zur Vernebelung und Mittel zur Steuerung ihrer Bewegung trägt, um es die Oberfläche am Boden befahren zu lassen, indem die zu behandelnde Umgebung durch Einteilung dieser Oberfläche in Gänge bedient wird, anhand einer autonomen Kartierung oder anhand von Daten, die durch Erlernen erworben werden.

## Claims

1. An apparatus for purifying an atmosphere to be treated comprising means for producing a fog by vibration of piezoelectric membranes, said apparatus including at least a battery (10) to mist a cleansing liquid equipped with piezoelectric membranes (11, 12), and means to convey various thus produced fog masses with a plurality of individualized airflows driving respectively the various fog masses apart from the membranes by individually guiding them towards a specific place of the atmosphere to be treated ; said apparatus including various cups in which various piezoelectric membranes of said misting battery are respectively provided and wherein are associated to it misted liquid flow control means acting by keeping a liquid level at a constant height above the corresponding membrane, said apparatus comprising a block (B1) integrating said misting battery and wherein are individually machined the various cups (111, 112) receiving the piezoelectric membranes as well as a plurality of internal cavities (121, 122) intended to collect the fog, each one opening into a fog guiding conduit rising towards the atmosphere to be treated by an airflow admitted in the corresponding cavities above said cups, **characterized in that** said apparatus comprises a tank (R, R') containing the cleansing liquid and means providing the recirculation towards said tank of liquid overflowing from the cups by outfall conduits (311, 312, 631, 632) ; the apparatus comprising a tank (R, R') containing the cleansing liquid, means to inject an airflow into said tank which include a fan (VA) located out of said block and said tank, and means to distribute this airflow (311, 312 ; 331, 332) towards said various cavities intended to collect the fog produced by the membranes in said cups ; said outfall conduits (311, 312) being connected on the one hand to lumens (123) machined within said block, respectively opening into said cavities, and on the other hand, to the upper part of said tank, said outfall conduits being further used as means to distribute said airflow injected into the tank.

2. The apparatus according to claim 1, **characterized in that** said guiding conduits (411, 412 ; 431, 432) for the individual guidance of the various fog masses, have a bent shape along an ascending path.

3. The apparatus according to claim 1 or 2, **characterized in that** it further comprises additional air blowing means (V1, V2 ; V3, V4) at the outlet of said fog guiding conduits, said means being provided outside of said block and so that the fog masses leaving out said conduits do not contact directly said means.

4. The apparatus according to one of claims 1 to 3, **characterized in that** it comprises a tank (R, R') containing the cleansing liquid and a weighing module (W) sensitive to the weight of said tank and means to determine the quantity of liquid consumed for the production of the fog masses over the time according to the tank weight determined by said weighing module.

5. The apparatus according to one of claims 1 to 4, **characterized in that** it comprises several misting batteries, each one being integrated into a machined block and each one being associated to several guiding conduits for ascending airflows, respectively, and a cleansing liquid feed tank common to said batteries.

6. The apparatus according to claim 5, comprising a movable frame (C) bearing said misting batteries and means intended to control its displacements to make him run the ground surface giving access to the atmosphere to be treated by partition of this surface into corridors from an autonomous cartography or data gathered by training.
